# EUROPÄISCHE PATENTANMELDUNG

(11) **EP 2 508 507 A1**
(43) Veröffentlichungstag der Anmeldung: **10.10.2012**
(21) Anmeldenummer: 11161507.6
(22) Anmeldetag: 07.04.2011
(51) Int. Cl.: C07C 69/30, C07C 407/00

(54) **Acylierte Glycerolverbindungen und deren Verwendung als Stabilisatoren bzw. Phlegmatisierungsmittel für Peroxide und Peroxidformulierungen**

(71) Anmelder: LANXESS Deutschland GmbH, 51369 Leverkusen (DE)
(72) Erfinder: Augustin, Thomas, 51065, Köln (DE)

(57) **Zusammenfassung**

Die vorliegende Erfindung betrifft neue acylierte Glycerolverbindungen die sich sehr gut als Stabilisator und/oder Phlegmatisierungsmittel für Peroxide und Peroxidformulierungen eignen.

## Beschreibung

Die vorliegende Erfindung betrifft neue Verbindungen auf Basis von Glycerolestern, und deren Verwendung als Stabilisator bzw. Phlegmatisierungsmittel für Peroxide und Peroxidformulierungen.

Peroxide sowie flüssige, pastöse oder feste Peroxidformulierungen können eingesetzt werden z.B. in Bleichmitteln, Reinigern, Desinfektionsmitteln, in Klebstoffen, Dichtstoffen oder Anstrichmitteln oder im klinischen Bereich als Sterilisierungsmittel sowie im Dentalbereich und in der Kosmetik. Desweiteren als Prozesschemikalien in der chemischen Industrie, als Radikalstarter bei Polymerisationen und in der Kautschukindustrie. Hier sind weiter zu nennen z.B. Crosslinker oder peroxidhärtende Systeme u.a. auch auf Basis von Cumylhydroperoxid oder Dibenzoylperoxid.

Peroxide und Peroxidformulierungen sind hochreaktive Verbindungen die meist sehr instabil sind und somit eine eingeschränkte Lagerstabilität aufweisen.

Aus dem Stand der Technik ist bekannt, dass zur Stabilisierung bzw. Phlegmatisierung von flüssigen, pastösen oder festen Peroxidformulierungen sowie wässrigen organischen peroxidhaltigen Emulsionen bzw. Dispersionen Weichmacher, wie Dialkyladipate und Phthalat-Weichmacher wie Dioctylphthalat (DOP) oder Phthalate, wie DEP, DBP oder DIBP, eingesetzt werden. Die Akzeptanz dieser Phthalatweichmacher wird immer häufiger in Frage gestellt. Insbesondere ist dies vor dem Hintergrund von REACH und der SVHC-Liste (Substances of very high concerns) zu sehen. Einige dieser Phthalate sind in der SVHC Liste bereits aufgeführt.

So beschreibt die WO 01/076936 A1 den Einsatz von Weichmachern auf Basis von längerkettigen Estern der Phthalsäure, längerkettigen Estern der Adipinsäure und mono-Carbonsäureestern zur Stabilisierung von wäßrigen, organische Peroxide enthaltenden Emulsionen.

Die u.a. in US-A-4,426,477 (vgl. auch US-A 2,633,456, US-A 3,390,115, US-A 3,578,621, US-A 4,072,790, US-A 4,073,782, US-A 4,143,011 und US-A 4,363,891) offenbarten Verbindungen auf Basis von Glycerolestern mit langen Acylresten finden ausschließlich ihren Einsatz als Weichmacher in der PVC Industrie. Diese bekannten Glycerolester können z.B. durch Acylierung von Glycerin hergestellt werden. Die hier beschriebenen Verbindungen umfassen Triester, wobei im Mittel zwei der Acylreste zwei Kohlenstoffatome enthalten und der verbleibende lange Acylrest mindestens 10 bis 14 Kohlenstoffatome enthält.

In US-2004/0106819 A1 wird hingegen die Herstellung von kürzerkettigen Glycerolestern als Modifikatoren für Polymere beschrieben.

Überraschenderweise wurden neue Verbindungen auf Basis von Glycerolestern, insbesondere auf Basis von Glycerolestern mit mittellangen Acylresten, gefunden, die zur Stabilisierung bzw. Phlegmatisierung von Peroxiden und flüssigen, pastösen oder festen Peroxidformulierungen geeignet sind.

Die vorliegende Erfindung betrifft neue Glycerolester mit mittellangen Acylresten, Verfahren zu deren Herstellung und deren Verwendung zur Stabilisierung und/oder Phlegmatisierung von Peroxiden und Peroxidformulierungen..

Gegenstand der vorliegenden Erfindung sind Verbindungen der Formel 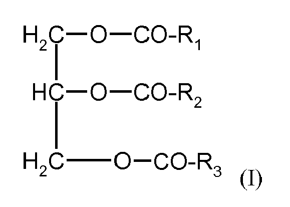 worin
R₁, R₂ und R₃ unabhängig voneinander für Wasserstoff oder einen Alkylrest mit 1 bis 9 C-Atomen stehen, mit der Maßgabe, dass wenigstens einer der Reste R₁, R₂ und R₃ für einen Alkylrest mit 1 bis 9 C-Atomen steht.

Bevorzugt sind Verbindungen der Formel (I), worin R₁, R₂ und R₃ unabhängig voneinander für Wasserstoff oder für einen Alkylrest mit 1 bis 8 C-Atomen stehen, mit der Maßgabe, dass wenigstens einer der Reste R₁, R₂ und R₃ für einen Alkylrest mit 1 bis 8 C-Atomen steht.

Besonders bevorzugt sind Verbindungen der Formel (I), worin R₁, R₂ und R₃ unabhängig voneinander für Methyl oder einen mittellangen Alkylrest mit 2 bis 9 C-Atomen stehen, mit der Maßgabe, dass wenigstens einer der Reste R₁, R₂ und R₃ für einen mittellangen Alkylrest mit 2 bis 9 C-Atomen und wenigstens einer der Reste R₁, R₂ und R₃ für Methyl steht.

Die beanspruchten Verbindungen umfassen Triester, wobei bevorzugt im Mittel zwei der Reste R1, R2 und R3, für Methyl stehen und einer der Rest, R1, R2 und R3 für einen Alkylrest mit 2 bis 9 Kohlenstoffatomen steht.

Ganz besonders bevorzugt sind Verbindungen der Formel (I), worin R₁, R₂ und R₃ unabhängig voneinander für einen mittellangen Alkylrest mit 2 bis 8 C-Atomen oder für Methyl stehen, mit der Maßgabe, dass wenigstens einer der Reste R₁, R₂ und R₃ für eine mittellangen Alkylrest mit 2 bis 8 C-Atomen und wenigstens einer der Reste R₁, R₂ und R₃ für Methyl steht.

Unter dem Begriff "mittellanger Alkylrest" oder "mittellanges Alkyl" ist ein Ethyl-, Propyl-, Butyl-, Pentyl, Hexyl-, Heptyl-, Octyl- und Nonylrest zu verstehen. Die als allgemein und bevorzugt genannten Alkylreste können sowohl geradkettig als auch verzweigt sein.

Weiterhin ganz besonders bevorzugt sind Verbindungen der Formel (I) worin zwei der Reste R₁, R₂ und R₃ für Methyl stehen und einer der Reste R₁, R₂ und R₃ für einen mittellangen Alkylrest mit 2 bis 9 C-Atomen, bevorzugt mit 2 bis 7 C-Atomen und ganz besonders bevorzugt mit 2, 3, 4, 5, oder 7 C-Atomen steht.

Weiterhin ganz besonders bevorzugt sind Verbindungen der Formel (I), worin R₁, R₂ und R₃ unabhängig voneinander für einen mittellangen Alkylrest mit 3 C-Atomen oder für Methyl stehen, mit der Maßgabe, dass wenigstens einer der Reste R₁, R₂ und R₃ für eine mittellangen Alkylrest mit 3 C-Atomen und wenigstens einer der Reste R₁, R₂ und R₃ für Methyl steht.

Weiterhin ganz besonders bevorzugt sind Verbindungen der Formel (I), worin R₁, R₂ und R₃ unabhängig voneinander für einen mittellangen Alkylrest mit 4 C-Atomen oder für Methyl stehen, mit der Maßgabe, dass wenigstens einer der Reste R₁, R₂ und R₃ für eine mittellangen Alkylrest mit 4 C-Atomen und wenigstens einer der Reste R₁, R₂ und R₃ für Methyl steht.

Weiterhin ganz besonders bevorzugt sind Verbindungen der Formel (I), worin R₁, R₂ und R₃ unabhängig voneinander für einen mittellangen Alkylrest mit 5 C-Atomen oder für Methyl stehen, mit der Maßgabe, dass wenigstens einer der Reste R₁, R₂ und R₃ für eine mittellangen Alkylrest mit 5 C-Atomen und wenigstens einer der Reste R₁, R₂ und R₃ für Methyl steht.

Weiterhin ganz besonders bevorzugt sind Verbindungen der Formel (I), worin R₁, R₂ und R₃ unabhängig voneinander für einen mittellangen Alkylrest mit 6 C-Atomen oder für Methyl stehen, mit der Maßgabe, dass wenigstens einer der Reste R₁, R₂ und R₃ für eine mittellangen Alkylrest mit 6 C-Atomen und wenigstens einer der Reste R₁, R₂ und R₃ für Methyl steht.

Weiterhin ganz besonders bevorzugt sind Verbindungen der Formel (I), worin R₁, R₂ und R₃ unabhängig voneinander für einen mittellangen Alkylrest mit 8 C-Atomen oder für Methyl stehen, mit der Maßgabe, dass wenigstens einer der Reste R₁, R₂ und R₃ für eine mittellangen Alkylrest mit 8 C-Atomen und wenigstens einer der Reste R₁, R₂ und R₃ für Methyl steht.

Insbesondere bevorzug sind Verbindungen der Formel

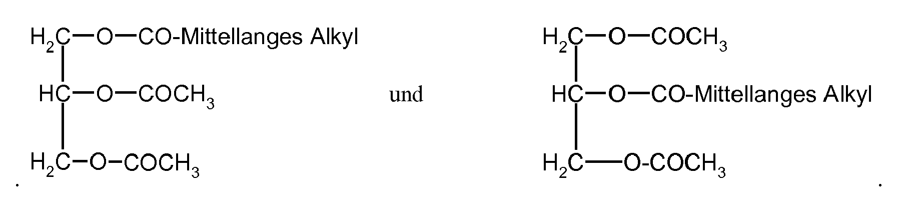

Herstellungsbedingt liegen die erfindungsgemäßen Verbindungen zumeist im Gemisch untereinander vor. Dabei ist der Methylrest im Allgemeinen in einer Menge von 0, 1.bis 2,9 Mol, bevorzugt von 1.0 bis 2.5 Mol und ganz besonders bevorzugt von 1,5 bis 2,5 Mol, und der mittellange Alkylrest im Allgemeine in einer Menge von 2,5 bis 0,5 Mol, bevorzugt von 1,5 bis 0,5 Mol und ganz besonders bevorzugt von 1,3 bis 0,8 Mol pro Mol Glycerin anwesend.

Das Mischungsverhältnis sowie die Verteilung der mittellangen Alkylreste und Methylgruppen im Molekül kann durch die Reaktionsführung bei der Herstellung beeinflusst werden. Eine typische Mischung hat folgende Zusammensetzung:
Hauptmenge 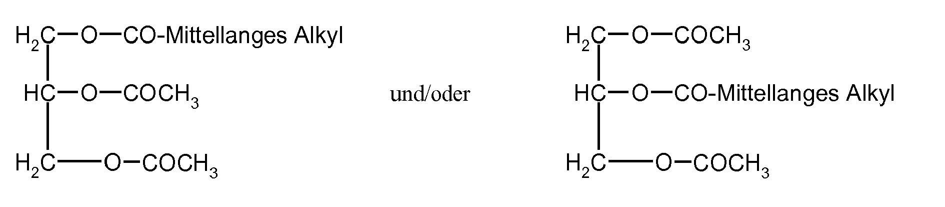
in geringem Umfang 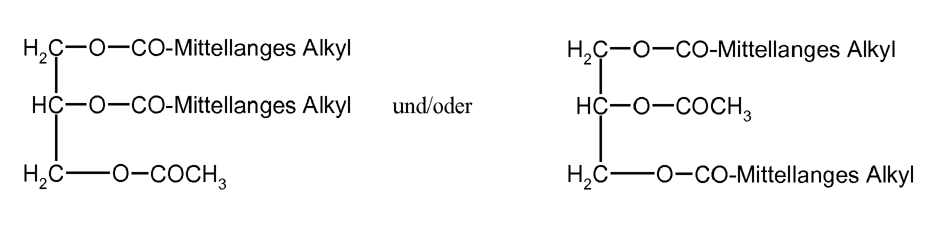 in sehr geringem Umfang

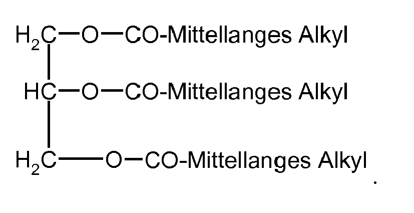

Die Verbindungen der Formel (I) sind neu. Ähnliche Verbindungen sind aus dem Stand der Technik bekannt (vgl. z.B. US-A-4,426,477 ). Die neuen Verbindungen der Formel (I) können prinzipiell in Analogie zu den bekannten Glycerolverbindungen in bekannter Art und Weise hergestellt werden. Im Allgemeinen erfolgt die Herstellung durch Reaktion von Glycerin mit Essigsäure und einer mittellangen Carbonsäure mit insgesamt 3 bis 10 C-Atomen bei einer Temperatur von 100 bis 130°C gegebenenfalls in Gegenwart eines Verdünnungsmittels und gegebenenfalls in Gegenwart von Katalysatoren, Säuren oder Basen. Zur Ausbeuteerhöhung kann in einem zweiten Schritt Essigsäureanhydrid nachgesetzt und die Reaktionstemperatur auf 160 bis 200°C erhöht werden. Nebenprodukte werden anschließend abdestilliert und das Endprodukt unter Vacuum abdestilliert.

Die vorliegende Erfindung betrifft weiterhin die Verwendung von mindestens einer Verbindung der Formel 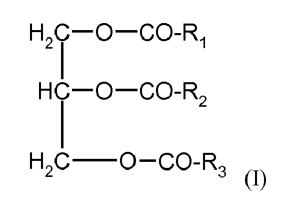 worin
R₁, R₂ und R₃ unabhängig voneinander für Wasserstoff oder einen Alkylrest mit 1 bis 9 C-Atomen stehen, mit der Maßgabe, dass wenigstens einer der Reste R₁, R₂ und R₃ für einen Alkylrest mit 1 bis 9 C-Atomen steht, zur Stabilisierung und/oder Phlegmatisierung von Peroxiden und/oder Peroxidformulierungen.

Bevorzugt ist die Verwendung von mindestens einer Verbindung der Formel (I), worin R₁, R₂ und R₃ unabhängig voneinander für Wasserstoff oder für einen Alkylrest mit 1 bis 8 C-Atomen stehen, mit der Maßgabe, dass wenigstens einer der Reste R₁, R₂ und R₃ für einen Alkylrest mit 1 bis 8 C-Atomen steht.

Besonders bevorzugt ist die Verwendung von mindestens einer Verbindung der Formel (I), worin R₁, R₂ und R₃ unabhängig voneinander für Methyl oder für einen mittellangen Alkylrest mit 2 bis 9 C-Atomen stehen, mit der Maßgabe, dass wenigstens einer der Reste R₁, R₂ und R₃ für einen mittellangen Alkylrest mit 2 bis 9 C-Atomen und wenigstens einer der Reste R₁, R₂ und R₃ für Methyl steht.

Bei den erfindungsgemäß zu verwendenden Triestern der Formel (I) stehen bevorzugt im Mittel zwei der Reste R1, R2 und R3, für Methyl und einer der Reste R1, R2 und R3 für einen Alkylrest mit 2 bis 9 Kohlenstoffatome.

Ganz besonders bevorzugt ist die Verwendung von mindestens einer Verbindung der Formel (I), worin R₁, R₂ und R₃ unabhängig voneinander für einen mittellangen Alkylrest mit 2 bis 8 C-Atomen oder für Methyl stehen, mit der Maßgabe, dass wenigstens einer der Reste R₁, R₂ und R₃ für eine mittellangen Alkylrest mit 2 bis 8 C-Atomen und wenigstens einer der Reste R₁, R₂ und R₃ für Methyl steht.

Weiterhin ganz besonders bevorzugt ist die Verwendung von mindestens einer Verbindung der Formel (I) worin zwei der Reste R₁, R₂ und R₃ für Methyl stehen und einer der Reste R₁, R₂ und R₃ für einen mittellangen Alkylrest mit 2 bis 9 C-Atomen, bevorzugt mit 2 bis 7 C-Atomen und ganz besonders bevorzugt mit 2, 3, 4, 5, oder 7 C-Atomen steht.

Weiterhin ganz besonders bevorzugt ist die Verwendung von mindestens einer Verbindung der Formel (I), worin R₁, R₂ und R₃ unabhängig voneinander für einen mittellangen Alkylrest mit 3 C-Atomen oder für Methyl stehen, mit der Maßgabe, dass wenigstens einer der Reste R₁, R₂ und R₃ für eine mittellangen Alkylrest mit 3 C-Atomen und wenigstens einer der Reste R₁, R₂ und R₃ für Methyl steht.

Weiterhin ganz besonders bevorzugt ist die Verwendung von mindestens einer Verbindung der Formel (I), worin R₁, R₂ und R₃ unabhängig voneinander für einen mittellangen Alkylrest mit 4 C-Atomen oder für Methyl stehen, mit der Maßgabe, dass wenigstens einer der Reste R₁, R₂ und R₃ für eine mittellangen Alkylrest mit 4 C-Atomen und wenigstens einer der Reste R₁, R₂ und R₃ für Methyl steht.

Weiterhin ganz besonders bevorzugt ist die Verwendung von mindestens einer Verbindung der Formel (I), worin R₁, R₂ und R₃ unabhängig voneinander für einen mittellangen Alkylrest mit 5 C-Atomen oder für Methyl stehen,
mit der Maßgabe, dass wenigstens einer der Reste R₁, R₂ und R₃ für eine mittellangen Alkylrest mit 5 C-Atomen und wenigstens einer der Reste R₁, R₂ und R₃ für Methyl steht.

Weiterhin ganz besonders bevorzugt ist die Verwendung von mindestens einer Verbindung der Formel (I), worin R₁, R₂ und R₃ unabhängig voneinander für einen mittellangen Alkylrest mit 6 C-Atomen oder für Methyl stehen, mit der Maßgabe, dass wenigstens einer der Reste R₁, R₂ und R₃ für eine mittellangen Alkylrest mit 6 C-Atomen und wenigstens einer der Reste R₁, R₂ und R₃ für Methyl steht.

Weiterhin ganz besonders bevorzugt ist die Verwendung von mindestens einer Verbindung der Formel (I), worin R₁, R₂ und R₃ unabhängig voneinander für einen mittellangen Alkylrest mit 8 C-Atomen oder für Methyl stehen, mit der Maßgabe, dass wenigstens einer der Reste R₁, R₂ und R₃ für eine mittellangen Alkylrest mit 8 C-Atomen und wenigstens einer der Reste R₁, R₂ und R₃ für Methyl steht.

Insbesondere bevorzug ist die Verwendung von Verbindungen der Formel 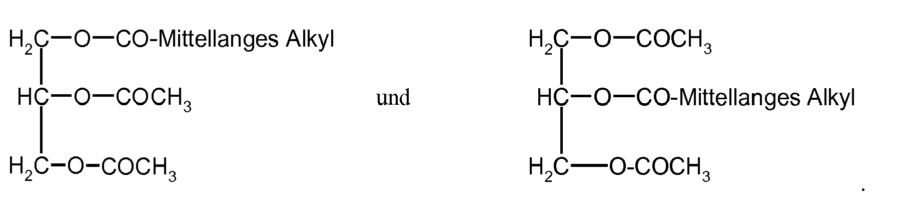 oder deren Mischung.

Ebenfalls bevorzugt ist die Verwendung von Mischungen der erfindungsgemäßen Verbindungen (I), wobei in der Mischung der Methylrest im Allgemeinen in einer Menge von 0,1.bis 2,9 Mol, bevorzugt von 1,0 bis 2,5 Mol und ganz besonders bevorzugt von 1,5 bis 2,5 Mol, und der mittellange Alkylrest im Allgemeine in einer Menge von 2,5 bis 0,5 Mol, bevorzugt von 1,5 bis 0,5 Mol und ganz besonders bevorzugt von 1,3 bis 0,8 Mol pro Mol Glycerin anwesend ist.

Die erfindungsgemäßen Verbindungen und deren Mischungen eignen sich hervorragend zur Stabilisierung und/oder Phlegmatisierung von Peroxiden und Peroxidformulierungen.

Überraschenderweise sind die erfindungsgemäß hergestellten Peroxide und Peroxidformulierungen stabil bzw. lagerstabil in dem Gebrauchszeitraum.

Weiterer Gegenstand der vorliegenden Erfindung sind Peroxide und Peroxidformulierungen enthaltend mindestens eine Verbindung der oben allgemein und bevorzugt angegebenen Formel (I).

Die erfindungsgemäßen Peroxide und Peroxidformulierungen enthalten im allgemeinen 0,1 bis 60 Gew.-%, bevorzugt 1,0 bis 50 Gew.-%, an mindestens einer Verbindung der Formel (I) bezogen auf die gesamte Formulierung.

Peroxidformulierungen im Sinne der Erfindung sind alle flüssigen, pastösen oder festen Zubereitungen von Peroxiden, als auch wässrige Peroxidlösungen. Der Gehalt an Wasserstoffperoxid kann von 0,1 bis 80 Gew.-% betragen.

Flüssige Peroxidformulierungen umfassen erfindungsgemäß insbesondere wässrige, organische Peroxide enthaltende Emulsionen bzw. Dispersionen. Solche organische Peroxide enthaltende Emulsionen oder Dispersionen enthalten üblicherweise wenigstens ein organisches Peroxid, Wasser, Antifrostmittel, chlorierte Kohlenwasserstoffe und gegebenenfalls oberflächenaktive Mittel und/oder Schutzkolloide.

Als organische Peroxide seien beispielhaft genannt: Hydroxyperoxide, Peroxyester, Peroxycarbonate, Peroxydicarbonate, Diacylperoxide, Dialkylperoxide und Bis-acylperoxyalkane.

Vorzugsweise seien genannt: Diisobutyrylperoxid, Cumylperoxyneodecanoat, 1,1,3,3-Tetramethylbutyl-peroxyneodecanoat, tert-Amyl-peroxyneodecanoat, tert-Butyl-peroxyneodecanoat, Dibutyl-peroxydicarbonat, 1,1,3,3-Tetramethylbutyl-peroxy-2-ethylhexanoat, 1,1,3,3-Tetramethylbutyl-peroxypivalat, tert-Butyl-peroxyneoheptanoat, tert-Amyl-peroxy-2-ethylhexanoat, tert-Amyl-peroxypivalat, tert-Butyl-peroxy-2-ethylhexanoat, tert-Butyl-peroxypivalat, tert-Butylperoxydiethylacetat, tert-Butyl-peroxyisobutyrat, Di(2-ethylhexyl)-peroxydicarbonat, Di(3,5,5-trimethylhexanoyl)peroxid, 2,5-Dimethyl-2,5-di(2-ethylhexanoylperoxy)hexan, 1-Hydroperoxy-1,3-dimethylbutyl-peroxypivalat, 1-(2-ethylhexylperoxy)-1,3-dimethylbutyl-peroxypivalat, 2-(2-Ethylhexanoylperoxy)-2-(pivaloylperoxy)-4-methylpentan und 2-(2-Ethylhexyloxycarbonylperoxy)-2-(isobutanoylperoxy)-5-methylhexan.

Die erfindungsgemäßen wässrigen, organische Peroxide enthaltenden Emulsionen oder Dispersionen enthalten üblicherweise 30 bis 70 Gew.-% an organischem Peroxid bezogen auf die gesamte Emulsion oder Dispersion.

Die erfindungsgemäßen Peroxidformulierungen können weitere Zusatzstoffe wie Schutzkolloide, Emulgatoren, UV-Stabilisatoren, Aktivatoren, Lösemittel, Trägersubstanzen, Harze oder andere Reaktivkomponenten enthalten. Diese genannten Zusatzstoffe sind dem Fachmann bekannt und werden den Peroxidformulierungen in üblichen Mengen zugesetzt.

Die erfindungsgemäßen Peroxidformulierungen können hergestellt werden indem mindestens eine Verbindung der Formel (I) sowie gegebenenfalls ein oder mehrere der oben genannten Zusatzstoffe den Peroxidformulierungen in einer Menge von 0,1bis 60 Gew.-% bezogen auf die Gesamtmenge zugesetzt werden.

Die Erfindung wird nachstehend anhand von Beispielen erläutert, es sei jedoch darauf hingewiesen, dass sie keineswegs auf diese Beispiele beschränkt ist.

### Herstellungsbeispiele

### I. Herstellung eines Propansäureglycerolesters

### Apparatur:

Mehrhalskolben, Mantelkolonne mit Berlsättelfüllung, Destillationsaufsatz mit Abnahmestelle, Kühler, Thermoelement, KPG-Rührer, Rührmotor.

### Ansatz:

1 Mol Glycerin, 1 Mol C3-Carbonsäure, 1,5 Mol Essigsäure und 1,2 Mol Essigsäureanhydrid.

### Durchführung:

Glycerin, Propansäure ( Propylsäure ) und Essigsäure wurden vorlegt und unter Stickstoff zum Rückfluß ( Sumpf : 110 - 125°C ) erwärmt bis sich die Temperaturen stabilisiert hatte (bei ca. 100°C). Nach ca. 1,5 Stunden wurde Wasser abdestilliert. Dann wurde die Sumpftemperatur langsam auf 180°C erhöht und solange destilliert bis die Kopftemperatur zusammenbrach. Anschließend wurde Essigsäureanhydrid innerhalb von 4 Std. zudosiert. Sobald sich die Kopftemperatur wieder stabilisiert hatte, wurde erneut destilliert, dabei kann die Kopftemperatur bis auf 120°C ansteigen. Anschließend wurde die Sumpftemperatur auf 120°C gesenkt und langsam evakuiert, die restliche Essigsäure wurde abdestilliert. Anschließend wurde die Sumpftemperatur langsam auf 180°C angehoben und das Produkt abdestilliert.

Das erhaltene Produkt kann mittels GC oder GC-MS charakterisiert werden

### II. Herstellung eines Butansäureglycerolesters

### Apparatur:

Mehrhalskolben, Mantelkolonne mit Berlsättelfüllung, Destillationsaufsatz mit Abnahmestelle, Kühler, Thermoelement, KPG-Rührer, Rührmotor.

### Ansatz:

1 Mol Glycerin, 1 Mol C4-Carbonsäure, 1,5 Mol Essigsäure und 1,2 Mol Essigsäureanhydrid.

### Durchführung:

Glycerin, Butansäure ( Butylsäure, Buttersäure ) und Essigsäure wurden vorlegt und unter Stickstoff zum Rückfluß ( Sumpf : 110 - 125°C ) erwärmt bis sich die Temperaturen stabilisiert hatte (ca. 100°C) . Nach ca. 1,5 Std. wurde Wasser abdestilliert. Dann wurde die Sumpftemperatur langsam auf 180°C erhöht und solange destilliert bis die Kopftemperatur zusammenbrach. Anschließend wurde Essigsäureanhydrid innerhalb von 4 Std. zudosiert. Sobald sich die Kopftemperatur wieder stabilisiert hatte, wurde erneut destilliert, dabei kann die Kopftemperatur bis auf 120°C ansteigen. Anschließend wurde die Sumpftemperatur auf 120°C gesenkt und langsam evakuiert, die restliche Essigsäure wurde abdestilliert. Anschließend wurde die Sumpftemperatur langsam auf 180°C angehoben und das Produkt abdestilliert.

Das erhaltene Produkt kann mittels GC oder GC-MS charakterisiert werden

### III. Herstellung eines Pentansäureglycerolesters

### Apparatur:

Mehrhalskolben, Mantelkolonne mit Berlsättelfüllung, Destillationsaufsatz mit Abnahmestelle, Kühler, Thermoelement, KPG-Rührer, Rührmotor.

### Ansatz:

1 Mol Glycerin, 1 Mol C5-Carbonsäure, 1,5 Mol Essigsäure und 1,2 Mol Essigsäureanhydrid.

### Durchführung:

Glycerin, Pentansäure ( Valeriansäure ) und Essigsäure wurden vorlegt und unter Stickstoff zum Rückfluß (Sumpf: 110 - 125°C) erwärmt bis sich die Temperaturen stabilisiert hatte (ca. 100°C) . Nach ca. 1,5 Std. wurde Wasser abdestilliert. Dann wurde die Sumpftemperatur langsam auf180°C erhöht und solange destilliert bis die Kopftemperatur zusammenbrach. Anschließend wurde Essigsäureanhydrid innerhalb von 4 Std. zudosiert. Sobald sich die Kopftemperatur wieder stabilisiert hatte, wurde erneut destilliert, dabei kann die Kopftemperatur bis auf 120°C ansteigen. Anschließend wurde die Sumpftemperatur auf 120°C gesenkt und langsam evakuiert, die restliche Essigsäure wurde abdestilliert. Anschließend wurde die Sumpftemperatur langsam auf 180°C angehoben und das Produkt abdestilliert.

Das erhaltene Produkt kann mittels GC oder GC-MS charakterisiert werden

### IV. Herstellung eines Hexansäureglycerolesters

### Apparatur:

Mehrhalskolben, Mantelkolonne mit Berlsättelfüllung, Destillationsaufsatz mit Abnahmestelle, Kühler, Thermoelement, KPG-Rührer, Rührmotor.

### Ansatz:

1 Mol Glycerin, 1 Mol C6-Carbonsäure, 1,5 Mol Essigsäure und 1,2 Mol Essigsäureanhydrid.

### Durchführung:

Glycerin, Hexansäure und Essigsäure wurden vorlegt und unter Stickstoff zum Rückfluß (Sumpf : 110 - 125°C) erwärmt bis sich die Temperaturen stabilisiert hatte (ca. 100°C) . Nach ca. 1,5 Stunden wurde Wasser abdestilliert. Dann wurde die Sumpftemperatur langsam auf 180°C erhöht und solange destilliert bis die Kopftemperatur zusammenbrach. Anschließend wurde Essigsäureanhydrid innerhalb von 4 Stunden zudosiert. Sobald sich die Kopftemperatur wieder stabilisiert hatte, wurde erneut destilliert, dabei kann die Kopftemperatur bis auf 120°C ansteigen. Anschließend wurde die Sumpftemperatur auf 120°C gesenkt und langsam evakuiert, die restliche Essigsäure wurde abdestilliert. Anschließend wurde die Sumpftemperatur langsam auf 180°C angehoben und das Produkt abdestilliert.

Das erhaltene Produkt kann mittels GC oder GC-MS charakterisiert werden

### V. Herstellung eines Oktansäureglycerolesters

### Apparatur:

Mehrhalskolben, Mantelkolonne mit Berlsättelfüllung, Destillationsaufsatz mit Abnahmestelle, Kühler, Thermoelement, KPG-Rührer, Rührmotor.

### Ansatz:

1 Mol Glycerin, 1 Mol C8-Carbonsäure, 1,5 Mol Essigsäure und 1,2 Mol Essigsäureanhydrid.

### Durchführung:

Glycerin, Oktansäure und Essigsäure wurden vorlegt und unter Stickstoff zum Rückfluß (Sumpf: 110 - 125°C) erwärmt bis sich die Temperaturen stabilisiert hatte (ca. 100°C) . Nach ca. 1,5 Stunden wurde Wasser abdestilliert. Dann wurde die Sumpftemperatur langsam auf 180°C erhöht und es wurde solange destilliert bis die Kopftemperatur zusammenbrach. Anschließend wurde Essigsäureanhydrid innerhalb von 4 Stunden zudosiert. Sobald sich die Kopftemperatur wieder stabilisiert hatte, wurde erneut destilliert, dabei kann die Kopftemperatur bis auf 120°C ansteigen. Anschließend wird die Sumpftemperatur auf 120°C gesenkt und langsam evakuiert, die restliche Essigsäure wurde abdestilliert. Anschließend wurde die Sumpftemperatur langsam auf 180°C angehoben und das Produkt abdestilliert.

Das erhaltene Produkt kann mittels GC oder GC-MS charakterisiert werden

### Anwendungsbeispiele

In den folgenden Beispielen wurden die nachstehend angegebenen erfindungsgemäßen Verbindungen gegenüber den aus dem Stand der Technik bekannten Weichmachern wie Dioctyladipat (DOA) und Alkylsulfonsäurephenolester (ASEP), C₁₂ -Fettsäure alcylierten Glycerindiacetat sowie Triacetin auf ihre stabilisierende Wirkung hin getestet. Überraschenderweise zeigen die erfindungsgemäßen Glycerolverbindungen eine deutlich verbesserte Stabilität bzw. Lagerstabilität.

### Beispiel 1

Verwendung von ASEP als Peroxidstabilisator

### • Keine Wirkung, nicht einarbeitbar

### Beispiel 2

Verwendung von Adipinsäureestem als Peroxidstabilisator

### • Keine Wirkung, nicht einarbeitbar

### Beispiel 3

Verwendung von Glycerintriacetat (Triacetin) als Peroxidstabilisator

### • Keine Wirkung

### Beispiel 4

Verwendung von einem C₁₂ Fettsäure alcylierten Glycerindiacetat ( Unimoll AGF).

### • Keine Wirkung, nicht verträglich, d.h. nicht mischbar.

### Beispiel 5

Verwendung eines C₃-Carbonsäure veresterten Glycerindiacetats gemäß Herstellungsbeispiel I.

### • Wirkung, d.h. langzeitstabil, keine Abnahme des Peroxidgehaltes

### Beispiel 6

Verwendung eines C₄-Carbonsäure veresterten Glycerindiacetats gemäß Herstellungsbeispiel II.

### • Wirkung, d.h. langzeitstabil, keine Abnahme des Peroxidgehaltes

### Beispiel 7

Verwendung eines C₅-Carbonsäure veresterten Glycerindiacetats gemäß Herstellungsbeispiel III.

### • Wirkung, d.h. langzeitstabil,. keine Abnahme des Peroxidgehaltes

### Beispiel 8

Verwendung eines C₆-Carbonsäure veresterten Glycerindiacetats gemäß Herstellungsbeispiel IV.

### • Wirkung, d.h. langzeitstabil, keine Abnahme des Peroxidgehaltes

### Beispiel 9

Verwendung eines C₈-Carbonsäure veresterten Glycerindiacetats gemäß Herstellungsbeispiel V.

### • Wirkung, d.h. langzeitstabil, keine Abnahme des Peroxidgehaltes

Die Wirksamkeit einer Verbindung wurde über die Entwicklung bzw. Stabilität der Viskosität und der Lagerstabilität der Formulierung über 50 Tage bestimmt.

## Patentansprüche

1. Verbindungen der Formel 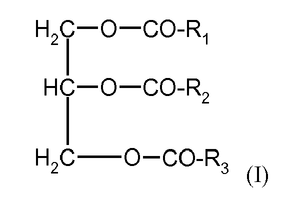 worin
R₁, R₂ und R₃ unabhängig voneinander für Wasserstoff oder einen Alkylrest mit 1 bis 9 C-Atomen stehen, mit der Maßgabe, dass wenigstens einer der Reste R₁, R₂ und R₃ für einen Alkylrest mit 1 bis 9 C-Atomen steht.

2. Verbindungen gemäß Anspruch 1, **dadurch gekennzeichnet, dass** in Formel (I) R₁, R₂ und R₃ unabhängig voneinander für Methyl oder einen mittellangen Alkylrest mit 2 bis 9 C-Atomen stehen,
mit der Maßgabe, dass wenigstens einer der Reste R₁, R₂ und R₃ für einen mittellangen Alkylrest mit 2 bis 9 C-Atomen und wenigstens einer der Reste R₁, R₂ und R₃ für Methyl steht.

3. Verbindungen gemäß Anspruch 1, **dadurch gekennzeichnet, dass** in Formel (I) R₁, R₂ und R₃ unabhängig voneinander für Wasserstoff oder für einen Alkylrest mit 1 bis 8 C-Atomen stehen, mit der Maßgabe, dass wenigstens einer der Reste R₁, R₂ und R₃ für einen Alkylrest mit 1 bis 8 C-Atomen steht.

4. Verbindungen gemäß wenigstens einem der Ansprüche 1 bis 3, **dadurch gekennzeichnet, dass** in Formel (I) R₁, R₂ und R₃ unabhängig voneinander für einen mittellangen Alkylrest mit 2 bis 8 C-Atomen oder für Methyl stehen,
mit der Maßgabe, dass wenigstens einer der Reste R₁, R₂ und R₃ für eine mittellangen Alkylrest mit 2 bis 8 C-Atomen und wenigstens einer der Reste R₁, R₂ und R₃ für Methyl steht.

5. Verbindungen gemäß wenigstens einem der Ansprüche 1 und 2, **dadurch gekennzeichnet, dass** in Formel (I), zwei der Reste R₁, R₂ und R₃ für Methyl stehen und einer der Reste R₁, R₂ und R₃ für einen mittellangen Alkylrest mit 2 bis 9 C-Atomen, bevorzugt mit 2 bis 7 C-Atomen und ganz besonders bevorzugt mit 2, 3, 4, 5, oder 7 C-Atomen steht.

6. Verbindungen gemäß wenigstens einem der Ansprüche 1, 2 und 5, **dadurch gekennzeichnet, dass** sie einer der Formeln 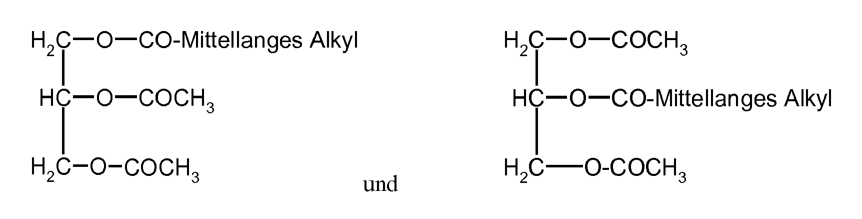 entsprechen, wobei "Mittellanges Alkyl" für Ethyl, Propyl, Butyl, Pentyl, Hexyl, Heptyl, Octyl oder Nonyl steht.

7. Verbindungen gemäß wenigstens einem der Ansprüche 1 bis 6, **dadurch gekennzeichnet, dass** der Methylrest im Allgemeinen in einer Menge von 0,1.bis 2,9 Mol, bevorzugt von 1.0 bis 2.5 Mol und ganz besonders bevorzugt von 1,5 bis 2,5 Mol, und der mittellange Alkylrest im Allgemeine in einer Menge von 2,5 bis 0,5 Mol, bevorzugt von 1,5 bis 0,5 Mol und ganz besonders bevorzugt von 1,3 bis 0,8 Mol pro Mol Glycerin anwesend ist.

8. Verfahren zur Herstellung von Verbindungen der Formel (I) gemäß Anspruch 1, **dadurch gekennzeichnet, dass** Glycerin mit Essigsäure oder Essigsäureanhydrid und einer mittellangen Carbonsäure mit insgesamt 3 bis 10 C-Atomen bei einer Temperatur von 110 bis 270°C gegebenenfalls in Gegenwart eines Verdünnungsmittels und gegebenenfalls in Gegenwart von Katalysatoren, Säuren oder Basen umgesetzt wird.

9. Verwendung von mindestens einer Verbindung der Formel 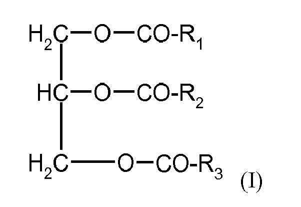 gemäß wenigstens einem der Ansprüche 1 bis 7,
zur Stabilisierung und/oder Phlegmatisierung von Peroxiden und Peroxidformulierungen, insbesondere Peroxidemulsionen und Peroxiddispersionen.

10. Peroxide und Peroxidformulierungen enthaltend mindestens eine Verbindung der Formel (I) 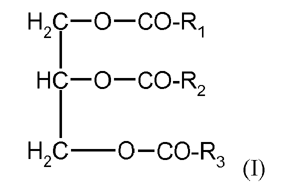 Gemäß wenigstens einem der Ansprüche 1 bis 7.
